# EUROPEAN PATENT APPLICATION

(11) **EP 3 951 022 A1**
(43) Date of publication of application: **09.02.2022**
(21) Application number: 20785336.7
(22) Date of filing: 02.04.2020
(51) Int. Cl.: C25F 1/08

(54) **PROCESS FOR MODIFYING THE SURFACE OF ELECTRODES FOR THE CONSTRUCTION OF ELECTROCHEMICAL BIOSENSORS**

(30) Priority: 02.04.2019 BR 102019006678
(71) Applicant: Universidade Federal De Uberlândia - Ufu, 38400-902 Uberlândia MG (BR); Cirino Alberto Goulart Eireli - EPP, 38400-170 Uberlândia - MG (BR)
(72) Inventor: GOULART FILHO, Luiz Ricardo, 38400-652 Uberlândia - MG (BR); BERNARDES GOULART, Isabela Maria, 38411-172 Uberlândia - MG (BR); NUNES RIELLO, Fabiane, 05366-000 São Paulo - SP (BR); OLIVEIRA NOTÓRIO, Ana Flávia, 38400-214 Uberlândia - MG (BR)
(74) Representative: Bosia, Alessandra
(86) International application number: PCT/BR2020/050112
(87) International publication number: WO 2020/198829

(57) **Abstract**

Development of a technique that is intended to modify, stabilize, functionalize, and reuse the surface of screen-printed electrodes, by means of the application of Rhodamine 6G as a working area modifying organic compound, enabling the creation of immunosensors that use proteins or their biological or synthetic fragments, antigens, antibodies, peptides, DNA, enzymes, RNA, and aptamers as analytes or as an element of biological recognition.

## Description

### Field of the invention

The invention concerns the development of a technique for stabilizing, functionalizing, and reusing the surface of screen-printed electrodes through the application of Rhodamine 6G (R6G) and the creation of an experimental model of electrochemical immunosensor for the detection of mycobacteria that can be exploited for the detection of other pathogens. Features of the invention caught the attention of scientific investments since the invention tries to overcome an eminent difficulty that is found in commercial electrodes, in which the lack of standardization and uniformity of the work area as well as the low level of recognition of biological samples with the surface make the use of such material impractical in the detection and diagnosis of diseases. Leprosy caused by *mycobacterium leprae* as well as other mycobacteria were used as a model for such application since it is a neglected tropical infectious disease that lacks a quick and sensitive differential diagnosis. R6G is the first organic compound used as a tool to stabilize, functionalize, and reuse the surface of commercial screen-printed electrodes. Its use can be exploited to detect any sample with protein and/or carbohydrate constitution.

### Background of the invention

### Biosensors

A biosensor is an integrated and self-sufficient device capable of providing analytical, quantitative, or semi-quantitative information using a biological recognition element (receptor) that guarantees specificity and produces a response that is translated by the physical component into an optical or electrical signal. In other words, the purpose of a biosensor is to produce a signal that is proportional, in magnitude or frequency, to the concentration of the analyte (NAKAMURA, H., KARUBE, I. Current research activity in Biosensors, Anal. Bional. Chem. 377: 446-468, 2003).

A receptor consists of a material of biological origin, such as: enzymes, organelles, animal or plant tissue, microorganism, antigen or antibody, nucleic acids, among others, which is immobilized on a support, connected to a base sensor (transducer) that converts the signal. (TRÉVENOT, D.R., TOTH, K., DURST, R. A., WILSON, G. S. Electrochemical biosensors: recommended definitions and classification. Biosensor & Bioelectronics. 16: 121-131, 2001). Biosensors can be classified as catalytic that generally use enzymes that are coupled to the working electrode. Such enzymes have a great activity combined with a high specificity for certain species, catalyzing the formation of electroactive products to be detected. Or affinity based on the formation of bonds between the analyte and the biological recognition component, by using biomolecules, wherein the binding essentially depends on the complementarity between the analyte and the active center of the biological component, either in size or shape, which allows high selectivity and sensitivity (PATACAS, R. C. Desenvolvimento, caracterização e optimização de um biossensor amperométrico para a determinação de Nitrato baseado em microinterfaces gelificadas. 123 f. Dissertation (Master's in Chemistry) - Science School of University of Porto, Porto, 2007).

It is possible to highlight the most used methods of immobilization: covalent bonding, physical adsorption, occlusion in gel or polymeric film that can form through simple immersion or application of electrical potential (electrodeposition). There are several biosensor application fields, which can be present in many sectors such as human and animal health, agriculture, food industry, pharmaceutical industry, the environment, the defense of the civilian population, in hospital infection control etc., since they have features that make them an increasingly attractive tool.

Among the desirable or ideal characteristics regarding a biosensor, we can highlight: selectivity, which is the ability to distinguish the molecule of interest from the others present in the sample; sensitivity, wherein the transducer is capable of converting a biological signal into an electrical signal proportional to the concentration level of the analyte in the sample; stability, time in which a biomolecule remains with its basic biorecognition characteristics; reproducibility, in which the experiment can be repeated by applying the same protocol, aiming to obtain results similar to the original; low cost and simplicity of operation (GALLI, A. Desenvolvimento e caracterização de um biossensor bioenzimático imobilizado sobre monocamadas auto-organizadas para determinação de açucares em alimentos. 143 f. Thesis (Doctoral in Sciences, Analytical Chemistry) - Chemistry Institute, University of São Paulo, São Carlos, 2009.) Biosensors can be classified according to the biolayer and the chosen transducer. Biolayers can be of the enzymatic type, which use enzymes as bioreceptor elements; cells, which use microorganisms, especially for the environmental monitoring of pollutants and immunosensors, which are based on the immunological reaction, with the antigen or antibody being the immobilized receptor on the surface of the sensor.

According to the chosen transducer, the biosensor can be classified as: optical, which are based on changes in optical properties of substances, which can be absorption, refractive index, fluorescence, phosphorescence, reflectivity and wavelength; piezoelectric, which detects the mass (related to the oscillation of the frequency of the piezoelectric crystals); calorimetric, which use the heat generated by catalyst reactions to measure the concentration of the analyte and where the biological element is translated into an electrical signal (DUTRA, RAF Desenvolvimento de biossensores em bioquímica clinica e imunodiagnóstico. Doctoral Thesis: Federal University of Pernambuco, 1999).

### Electrochemical Biosensor

Electrochemical biosensor is probably the largest class of chemical sensors, being used in the detection of the most diverse molecules, microorganisms etc. Most transducers used in commercial biosensors are of this type.

They can be divided into potentiometric, amperometric, voltammetric, conductometric and impedometric. Amperometrics and potentiometrics have been the most used. Amperometrics are based on current intensity measurements in an electrochemical cell at a fixed potential, the current being generated by oxidation reaction or electrochemical reduction of electroactive species on the sensitive surface, proportional to the analyte concentration. In this technique, the working electrode potential is usually kept constant in relation to the reference electrode. Unlike amperometrics, voltammetric biosensors work with a potential variation for the detection system until oxidation or reduction of the analyte occurs, with a variation in the peak current upwards or downwards. In potentiometric measurements, the magnitude of the potential measured at near-zero currents is due to the potential difference between an indicator electrode and a reference electrode. Conductometric biosensors measure the conductance between a pair of metallic electrodes, resulting in the production of ionic species, resulting from the interaction of an enzyme with the analyte. Finally, impedimetric biosensors are those that use electrochemical impedance measurements to detect the antigen-antibody interaction on the modified surface of the electrode. (TRÉVENOT, DR; TOTH, K.; DURST, RA; WILSON, GS Electrochemical biosensors: recommended definitions and classification. Biosensor & Bioelectronics. 16: 121-131, 2001 / MOHANTY, SP, KOUGIANOS, E. Biosensors: the tutorial review review. IEEE Potentials, 25(2): 35-40, 2006).

Electrochemical detection is based on two principles, direct and indirect. Direct detection is based on the principles of oxidation of nitrogenous bases (in the case of DNA) and amino acids (in the case of peptides and proteins), in which, from specific potentials, quantitative values can provide the concentration of these biomolecules (WO 2012153124 A1 / WO2009045116 A1). Indirect detection uses electrochemical indicators, which are susceptible to oxidation and/or reduction, also with proportional quantitative values (directly or inversely proportional).

### Electrodes

The choice of the surface where the bioreactions (electrodes) will occur is a fundamental part in the process of creating biosensors. Electrochemical sensors with disposable electrodes have been an interesting option for application in various types of systems, since they can be mass produced, at a low cost and without some limitations presented by solid electrodes, such as the lack of repeatability of the active area between successive polishing and the difficulty of regeneration of the surface after use, resulting in greater cost in execution (NASCIMENTO, V. B. ANGNES, L. Eletrodos fabricados por "silk-screen". Quím. Nova. 21(5): 614-629, 1998).

The electrodes printed by screen printing system are called SPE (Screen Printed Electrode) or silk-screen. It has been used with great success for enabling mass production at extremely low cost, simplicity of use, high sensitivity, selectivity, precision, stability, and quick responses, in addition to being disposable. SPEs consist of a film deposited on a substrate that will serve as a support and provide a surface for the electrode to be printed, usually of PVC or alumina ceramic. It is important that the substrate material is thermally and chemically inert, with low residual current and high electrical conductivity capable of resisting the signal generation produced by the biosensors. Then, the inks are deposited on the substrate, and they can be conductive and dielectric. In conductive paints, the material used can be metal powders such as gold, platinum, silver, palladium, and carbon, which are dispersed in the binder. Carbon has been widely used as a raw material for making electrodes due to its characteristics such as high chemical inertia and electrochemical inertia, the latter in a wide range of potentials; low current residual; high electrical conductivity and for providing easy surface regeneration. In general, such film is partially covered by a second layer of an insulator to define an electrical contact area at one end and another area to be the electrode surface. (BRAININA, K.H. Z., BOND, A. M., Anal. Chem. 67: 2586, 1995 / COOPER, J. M., CASS, A. E. G. Biosensors. 2nd ed. Oxford University Press.United States. 268 f., 2004).

Printed electrodes have the advantage of being disposable, in addition to the ease of miniaturization, since, due to its planar format, it provides its integration to small portable devices. The integrated three electrode system - working electrode (ET); reference electrode (RE) and auxiliary electrode (EA) enable its use in small portable equipment.

### Modified electrodes

Screen-printed electrodes, despite being applied in many situations, have a limitation, since in most cases they require surface modification aiming mainly to control the reactions that occur at the interface and providing greater selectivity for a given analyte. The great difficulty with these commercial electrodes is the lack of standardization of the work area, even in electrodes made on the same production line, as well as the difficulty of adherence of some biomolecules to the work area surface. And despite being disposable, there is a higher cost for not being reused.

Modified electrodes are defined as those that have immobilized, on their surfaces, chemically active species such as film or monomolecular, multimolecular, ionic or polymeric layer aiming to control the physical-chemical properties of the electrode-solution interface (EDWARDS, G. A., BERGREN, A. J., PORTER, M. D. Chemically Modified Electrodes. In: ZOSKI, C. G. Handbook of Electrochemistry. Elsevier. 295-327, 2007).

This combination between the versatility of printed electrodes and the possibility of modifying their surface with specific reagents, has significantly expanded their applications. There are, in the literature, modified electrodes in the most diverse ways, such as with mercury film (WANG, J. Decentralized electrochemical monitoring of trace-metals-from disposable strips to remote electrodes - plenary lecture. Analyst. 119: 763- 766, 1994), bismuth film (WANG, Z., WANG, H., ZHANG, Z., LIU, G. Electrochemical determination of lead and cadmium in rice by a disposable bismuth/electrochemically reduced grapheme/ ionic liquid composite modified screen-printed electrode. Sensors and Actuators B. 199:7-14, 2014), lead film (BOBROWSKI, A., KROLICKA, A., MACZUGA, M., ZAREBSKI, J. A novel screen-printed electrode modified with lead film for adsorptive stripping voltammetric determination of cobalt and nickel. Sensors and Actuators B. 191: 291-297, 2014), gold nanoparticles (ASADOLALLAHI-BABOLI, M. MANI-VARNOSFADERANI, A. Rapid and simultaneous determination of tetracycline and cefixime by mean of gold- nanoparticles-screen-printed gold electrodes and chemometrics tools. Measurements. 47: 145-149, 2014), nickel oxide nanoparticles (RAFIEE, B., FAKHARI, A. R. Electrocatalytic oxidation and determination of insulin at nickel oxide nanoparticles-multiwalled carbon nanotube modified screen printed electrode. Biosensors and Bioelectronics. 46: 130-135, 2013), and polymeric films, which are also widely used for this purpose (PI 0413769-8 / BR 102016018862-8).

### Rhodamine

Rhodamines are a family of organic chemicals used as dyes, known as fluorones, and are a fluorescent cationic compound of formula C₂₇H₂₉ClN₂O₃. Such dyes have long been used as tracking agents in studies of water, pollution and spraying of airborne pesticides, as well as in the coloring of drugs, cosmetics, textiles, and paints. These compounds are widely used in biotechnological applications such as fluorescence microscopy, flow cytometry, fluorescence spectroscopy and ELISA (International Agency for Research on Cancer. Rhodamine B. IARC Monogr., 16: 221-231. 1978).

Rhodamine 6G is also known as Rhodamine 590, R6G, Rh6G, Basic Red 1 or Basic Rhodamine Yellow. Under normal pressure and temperature, this compound is a dark purple-red, brown, or black crystalline solid. It has a molar mass of 479.02 g mol-1 and a density of 1.26. It is highly soluble in water and many organic solvents. Although very soluble, this form is quite corrosive to all metals and alloys except stainless steel. Other formulas are less soluble but less corrosive. It has great photostability and a high fluorescence quantum yield (0.95) and has a maximum absorption at 530 nanometers. This dye is produced from the condensation of 3-ethylamino-p-cresol (3-ethylamino-4-methylphenol) with phthalic anhydride, followed by ethanol esterification by acid catalysis (GEORGES, J., ARNAUD, N., PARISE, L. Limitations arising from optical saturation in fluorescence and thermal lens spectrometries using pulsed laser excitation: Application to the determination of the fluorescence quantum yield of rhodamine 6G. Applied Spectroscopy. 50(12): 1505-1511., 1996 / PUBCHEM. Available in: <https:pubchem.ncbi.nlm.nih.gov/compound/rhodamine_6g#section=Top>. Accessed on: 08/14/2018).

Typically, cationic dyes of this type are used in lasers and as probe molecules in various biological applications, mainly due to their low toxicity, quantum efficiency, small dependence on environmental factors, and their fluorescent characteristics do not change over several if the aqueous solution is continuously stirred. (ARCOUMANIS, C., MCGUIRK, J.J., PALMA, J.M.L.M. On the use of fluorescent dyes for concentration measurements in water flows, ed. E.i. Fluids, 1990).

In genosensors, it is common to use chemical compounds as indicators to prove the process of selective hybridization of DNA, RNA and aptamers as shown in PI 9302052-0.

Compounds that have aromatic rings can be used as electrochemical indicators since they interact with DNA through electrostatic bonding or fusion (RICHARDS, A. D., RODGERS, A. Synthetic metallomolecules as agents for the control of DNA structure. Chemical Rev. 36(3): 471-483, 2007).

Several studies report methylene blue, ethidium bromide and tetramethylbenzidine as DNA indicators (ALVES-BALVEDI, R.P., CAETANO, L.P., MADURRO, J.M., BRITO-MADURRO, A.G. Use of 3,3',5,5'tetramethylbenzidine as new electrochemical indicator of DNA hybridization and its application in genossensor. Biosens. Bioelectron. 85: 226-231, 2016 / BALVEDI, R.P.A., CASTRO, A.C.H., MADURRO, J.M., BRITO-MADURRO, A.G., Detection of a Specific Biomarker for Epstein-Barr Virus Using a Polymer-Based Genosensor. Int. J. Mol. Sci. 15: 9051-9066, 2014 / TALEATAT, Z., CRISTEA, C., MARRAZZA, G., MAZLOUM-ARDAKANI, M.,

SANDULECU, R. Electrochemical immunoassay based on aptamer-protein interaction and functionalized polymer for cancer biomarker detection. J. Electroanalyt. Chem. 15: 119-124, 2014).

In research with electrochemical indicators, to indicate the biological recognition between proteins, BR shows the application of tetramethylbenzidine (4-DMAA) since it is an organic compound that can oxidize, reduce and interact with biomolecules.

The process of industrial synthesis and preparation of rhodamines (B, 3B, 6G and others) is described in EP 0468821 A1 and the same process for Florina, a rhodamine with radioisotope F18, is described in US 9101673 B2.

The preparation of concentrated and diluted solutions, stable, aqueous or organic solvents of R6G (C₂₈H₃₁N₂O₃Cl, MM 479,02g/mol; solubility in water 20 g/l and in methanol 400 g/l) are contained in US 20070531413 / WO2007RU00130 / US 6191278 B1 / WO 2005007678 A2.

Patent US 3849065 A describes how dye solutions such as rhodamine esterified with glycol and/or glycol derivative would be used to dye paper.

Patent US 6750357 B1 shows that rhodamine can be used as a raw material for the synthesis of other compounds excitable by light at appropriate wavelengths. Application of public health interest was the use of rhodamine in the treatment of cancer cells, as described in patents WO2010IL00233 / US2010144854.

The interaction of different types of rhodamines in DNA sequencing process is described in US 5366860 A.

In electrochemical analysis, rhodamine is used in the detection of metal ions (KAMAL, A., KUMAR, N., BHALLA, V., Rhodamine-dimethyliminocinnamyl based electrochemical sensors for selective detection of iron (II). Sens Act B Chem. 190: 127-133, 2013 / KIM, H., LEE, D-H., SON, Y-A. Electrochemical Study on Rhodamine 6G-Indole Based Dye for HOMO and LUMO Energy Levels. Text. Coloration Finish. 25: 7-12, 2013).

Rhodamine 6G is widely used in Ramam spectroscopy as an analyte model to test modified surfaces as in the case of modification with silver nitrate to build a biosensor for colorectal cancer and in surfaces coated with gold nanostructures due to detectable signals of fluorescence emitted by said dye (ORSAGOVA, K. Z., ORIŇAK, A., ORIŇAKOVA, R., PETRU 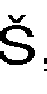 , O., MACKO, J., RADONAK, J., SUKOVSKA, L. L., JURA 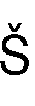 EKOVA, Z., SMITH, R., STRECKOVA, M., KOVAL, K. Electrochemically deposited silver detection substrate for surface-enhanced Raman spectroscopy cancer diagnostics. J Biomed Opt. 23(7): 1-11. 2018 / TRAN, M., FALLATAH, A, WHALE, A., PADALKAR, S. Utilization of Inexpensive Carbon-Based Substrates as Platforms for Sensing. Sensors (Basel). 27:18(8), 2018).

Rhodamine 6G is an interesting technological application molecule and is commonly used in microscopy, flow cytometry, spectroscopy, ELISA and Ramam sensors. But no uses of the dye in electrochemical biosensors or in the preparation of surfaces to stabilize and bind biomolecules on electrodes were found.

With the aforementioned analyses, Rhodamine 6G was tested as a surface modifier of the working area of screen-printed commercial electrodes as it presents aromatic rings binding carbohydrates and a structure with affinity to biological molecules such as proteins, thus making the surface more receptive to biological elements such as antigens and antibodies, being used for preparation of biological sensing techniques and disease diagnosis.

### Leprosy

Leprosy is a chronic infectious disease caused by *Mycobacterium leprae,* affecting mainly the skin and peripheral nerves (WALKER, S.L., LOCKWOOD, D.N.J. Leprosy. Clin. Dermatol. 25(2): 165-172, 2007).

In 2015, the World Health Organization registered 211,973 new cases of leprosy, with 26,395 only in Brazil, which makes it the second country with the highest prevalence of leprosy in the world (**WORLD HEALTH ORGANIZATION.** Leprosy elimination. Available at: <http://www.who.int/lep/en/>. Accessed on: 11.01.2016). In addition, leprosy is a mandatory notification disease in Brazil and requires mandatory investigation.

*M. leprae* diagnosis is based mainly on clinical tests performed in dermato-neurological examination. (DUTHIE, M.S., HAY, M.N., RADA, E.M., CONVIT, J., ITO, L. Specific IgG antibody responses may be used to monitor leprosy treatment efficacy and as recurrence prognostic markers. Eur. J. Clin. Microbiol. Infect. Dis. 30: 1257-1265, 2011).

Currently, complementary laboratory tests have demonstrated an important tool in the diagnosis of leprosy. The polymerase chain reaction (PCR) technique has good sensitivity in multibacillary leprosy, but it is generally poorly sensitive (less than 50%) in cases of paucibacillary leprosy. In addition, PCR technology requires high-cost equipment and infrastructure, as these are tests for differential and complementary diagnosis, not being used in the routine of primary care services, only in reference services and in research (SANTOS, A. R., MIRANDA, A. B., SARNO, E. M., SUFFYS, P. N., DEGRAVE, W.M. Uso de PCR mediada por amplificação de Mycobacterium leprae DNA em diferentes tipos de amostras clinicas para o diagnóstico da lepra. J. Med. Microbiol. 39: 298-304, 1993). Therefore, there is a growing interest in the development of portable, simple, fast, and accurate methods for detecting such pathogens. Methods involving electrochemical detection have shown new possibilities for diagnosing infectious diseases or other diseases.

Due to the search for new diagnostic techniques for infectious diseases such as leprosy and tuberculosis, these diseases were then used as an experimental model to test the use of electrodes modified with rhodamine 6G.

### Description and detailing of the components of the invention

Screen-printed commercial electrodes are widely used in the creation of electrochemical sensors, but their use is limited due to the lack of standardization of the work area and little recognition with biological samples, thus requiring modifying agents. Aiming to provide a low-cost, low-toxicity surface modifier that is efficient, stable and with affinity to biological molecules, the invention presents the modification of the transducer surface of a commercial screen-printed electrode with rhodamine 6G, a dye capable of stabilizing and homogenizing the surface and increase the active area of the electrodes ensuring greater passage of electrons and greater biological recognition by having benzene rings in its structure that are capable of interacting with DNA and RNA through electrostatic binding or fusion, enabling the biosensor in the recognition and diagnosis of these samples.

In this platform, antibodies specific for some bacteria were used, immobilized on the transducer surface of a commercial screen-printed electrode modified with rhodamine 6G as a biological recognition component, however, it should be noted that this application serves as an example of the feasibility of its use, not being exclusively intended for this purpose, but being intended for use in the diagnosis of other diseases and/or recognition of biomolecules that have proteins or their biological or synthetic fragments , antigens, antibodies, peptides, enzymes DNA, RNA and aptamers.

The application results in the construction of an immunosensor that uses a screen-printed DRP110 graphite electrode as a conductive material and rhodamine 6G dye as a work area modifier and electrochemical analysis, following the variation of signals by differential pulse voltammetry, cyclic voltammetry, or other appropriate electrochemical technique, of the oxidation peak or reduction of specific recognition binding between probe and targets. For specificity and sensitivity assays of the modified immunosensor, specific antibodies, their respective native or mimetic antigens, bacterial cultures, and clinical samples of dermal scrapings from patients were used. However, the present invention is not restricted only to the use for these samples, but for immunosensors in general.

The chosen electrode must be made of a preferably conductive material and present electrochemical inertia in the range of -0,4 V to +1,4 V (versus Ag/AgCl or Ag), such as graphite, glassy carbon, paste carbon, diamond, gold, platinum or even combinations thereof. Said materials and biological recognition compounds can also be incorporated into nanotechnological materials such as polymeric films, graphene, carbon nanotubes and nanoparticles, aiming at increasing conductivity and improving electrochemical signals.

### Description of figures

For a better understanding of the characteristics of the present invention, which uses rhodamine 6G as a modifier of the transducer surface of a commercial screen-printed electrode, exemplifying graphic results are presented which represent a way of producing the immunosensor for the diagnosis of mycobacteria such as leprosy and tuberculosis, by way of example of the invention.

### Interaction of Rhodamine 6G with the electrode surface

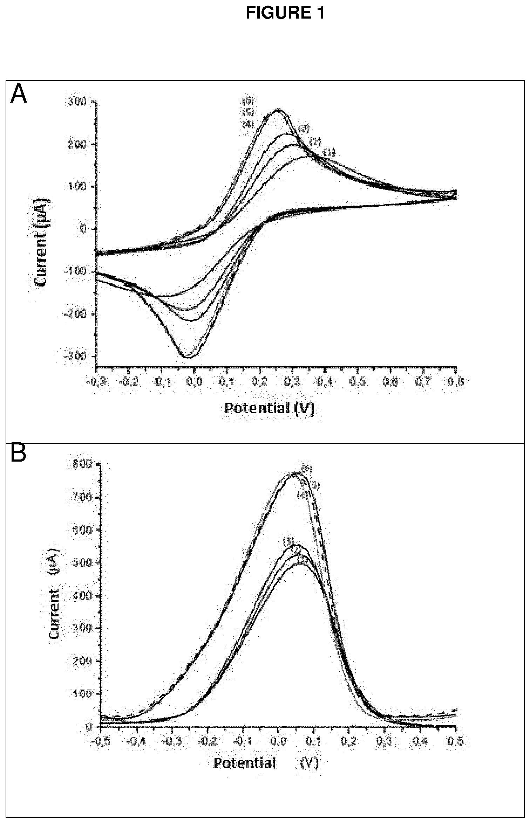

FIGURE 1 illustrates the cyclic voltammetry (VC) scan (FIG. 1A) and the differential pulse voltammetry (VPD) (FIG. 1B) for three different electrodes modified with rhodamine 6G (4.5 and 6), and the same electrodes before adsorption of this component (1, 2 and 3). It is observed that in the electrode voltammetries without modification there was a great variation, over 50uA, in the oxidation current of VPD and VC, and the potential also varied from 0.2 to 0.4V. While in the modified electrodes with rhodamine 6G, in addition to a significant increase in current peaks in VC and VPD, indicating greater electron passage, the currents and potentials of these modified electrodes remained very homogeneous between the voltammetric analysis between different electrode areas. Rhodamine 6G has efficient adsorption on carbon, possibly due to Van der Waals interactions between the planar benzene ring and the electrode surface. Thus, it was proved that this modification was useful to homogenize, standardize and increase the conductivity of the transducer surface of the electrodes, preparing them for sample application.

### Investigation of rhodamine 6G concentration

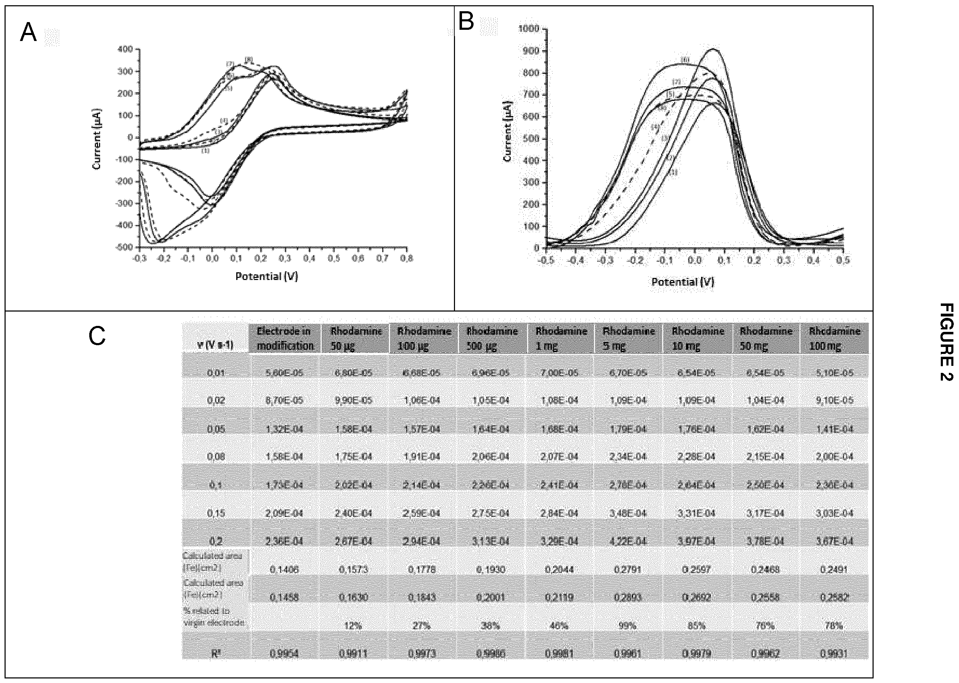

FIGURE 2 shows the adsorption of different concentrations of rhodamine 6G on the surface of the electrodes, in order to study their interaction to choose the best dilution to be used in the next tests. FIG 2A and B show cyclic and differential pulse voltammograms for the following rhodamine dilutions: (1) 50µg, (2) 100µg, (3) 500µg, (4) 1mg, (5) 5mg, (6) 10mg, (7) 50mg, (8) 100mg. Through the VC analysis we observed that as the rhodamine concentration increases, the oxidation and reduction current also increases, however, the design of the voltammetric curves are distorted and the peaks start to become more spaced from the concentration of 1mg, probably due to the accumulation and saturation of rhodamine on the electrodes surface. In the VPD in FIG 2B, the oxidation peak decreases with a concentration above 500µg, proving once again that this is the limit concentration to efficiently modify the electrodes, and that concentrations above this margin saturate the transducer surface, making it difficult to bind probes and targets. FIG 2C shows a table with the values of scan speeds of the peaks of the oxidation currents in cyclic voltammetry for different studied concentrations of rhodamine. These values were used to formulate the Randles-Sevcik equation that measures the active area and the passage of electrons on the transducer surface, thus showing that the modification of electrodes with rhodamine increases up to 99% of the active area in relation to unmodified electrodes.

### Detection of antigens with modified electrodes

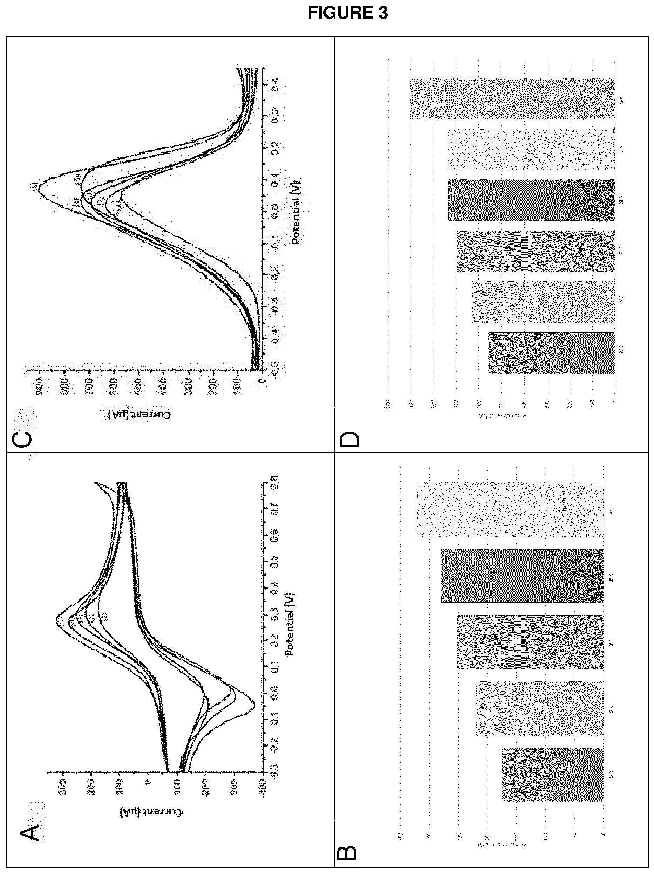

For the construction of the bioelectrode, the specific antibody for *micobactérium leprae* Anti-PGL-1 antigens was immobilized on the surface of the electrodes modified with rhodamine 6G, and recognition was tested with some mimetic, synthetic and native antigens that specifically recognize this probe. In FIGURE 3A, cyclic voltammetry was performed to determine the difference between the binding of the antibody with the PGL-1 native antigen (curve 1), the antibody with the M3R mimetic antigen (curve 5) and the antibody without recognition antigen (curve 3), curve 2, electrode without modification and curve 4, electrode modified with rhodamine, but without having the targets serving as baselines.

Figure 3B presents the bar graph of the same analysis as above, with the values of the currents of the oxidation peaks of the connection in each electrode at the potential of 0.25V. Analyzing the current peaks at this potential, it is possible to verify the difference when there is antibody binding to the antigens and when there is no recognition. In FIGURE 3C, differential pulse voltammetry analyzes were performed with the same anti-PGL-1 antibody recognizing other specific antigens for comparison. CURVE 1 represents the binding of the antibody with the native PGL-1 antigen, in CURVE 2 the antibody is without recognition antigen, in CURVE 3 the antibody is recognizing the LAM antigen of M. *leprae,* in CURVE 4 the binding is with the synthetic PGL-1 antigen, in CURVE 5 with mimetic PGL-1 antigen, the M3R, and in CURVE 6 the antibody recognized another mimetic antigen, MPML14. In FIGURE 3C the same connections were analyzed by the bar graph.

It can be concluded with such analyzes that each type of antigen has a specific recognition, and that all curves differ from the curve without the recognition target, showing the specificity of the constructed bioelectrode.

### Investigation of bioelectrode cleaning and reuse

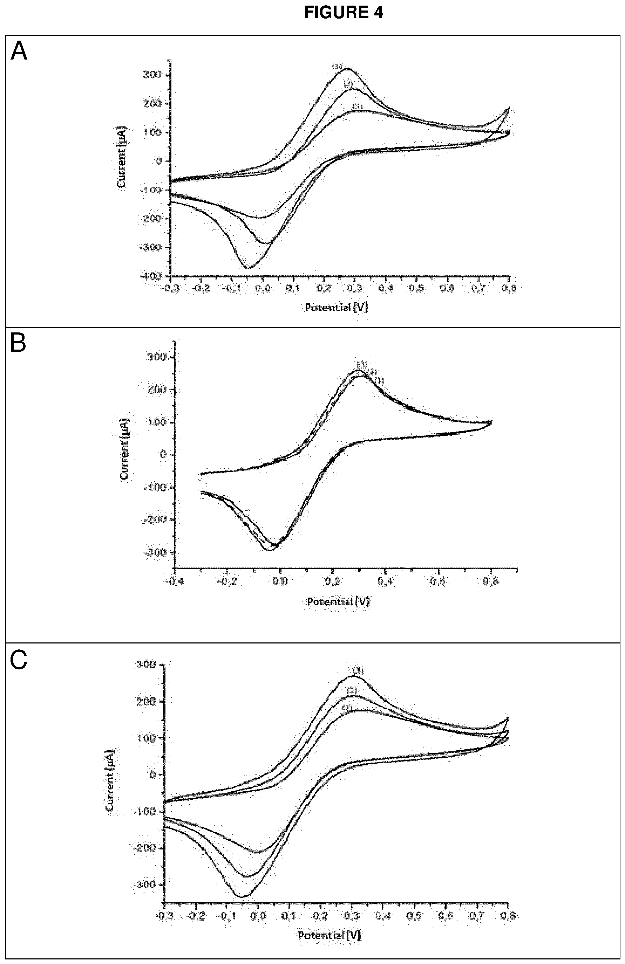

FIGURE 4 shows the cleaning and reuse of the bioelectrode modified with rhodamine 6G, even after adsorption of antibody and antigens In FIG. 4A we have the cyclic voltammograms of the modified electrodes immobilized with the Anti-PGL-1 antibody with native PGL-1 antigen (curve 1), without recognition antigen (curve 2), and with M3R mimetic antigen (curve 3). After these readings and curve differentiation, the electrodes were washed with alcohol and reused for new adsorptions. In FIG. 4B, the electrodes that were washed and returned to their original baseline were modified again with rhodamine 6G, and it is possible to note that curves that were different in the previous figure due to the immobilizations were homogeneous, proving the cleanliness of the electrode with alcohol and homogeneity of the surface with the deposition of rhodamine. The electrodes modified for the second time were then immobilized with the same antibody, but the order of adsorption of the antigens was modified. The electrode that had not been immobilized with antigen was immobilized with native PGL-1 antigen (FIG. 4C curve 1) in the second test, the electrode that had been immobilized with M3R antigen had no added antigen (FIG. 4C curve 2) in the second test, and the electrode that had been adsorbed with native PGL-1 antigen in the first modification, had the M3R antigen added in the second immobilization. Even after being washed and used in immobilizations on different electrodes, the bioelectrode maintained its ability to differentiate antigens with the oxidation and reduction peaks remaining very close to the modified and immobilized electrodes for the first time. This study demonstrated that electrodes modified with rhodamine 6G have the ability to be cleaned and reused even after adsorption of probes and targets, as they bind to the chemical compound previously adsorbed and not directly on the electrode surface, and when the modified bioelectrode is washed with some organic solvent, alcohol in the case of the present test, it manages to undo the bonds of the benzene ring between the rhodamine and the carbon on the electrode surface, washing together all the biological material that had been deposited on the rhodamine.

### Prototype for differentiating between clinical samples

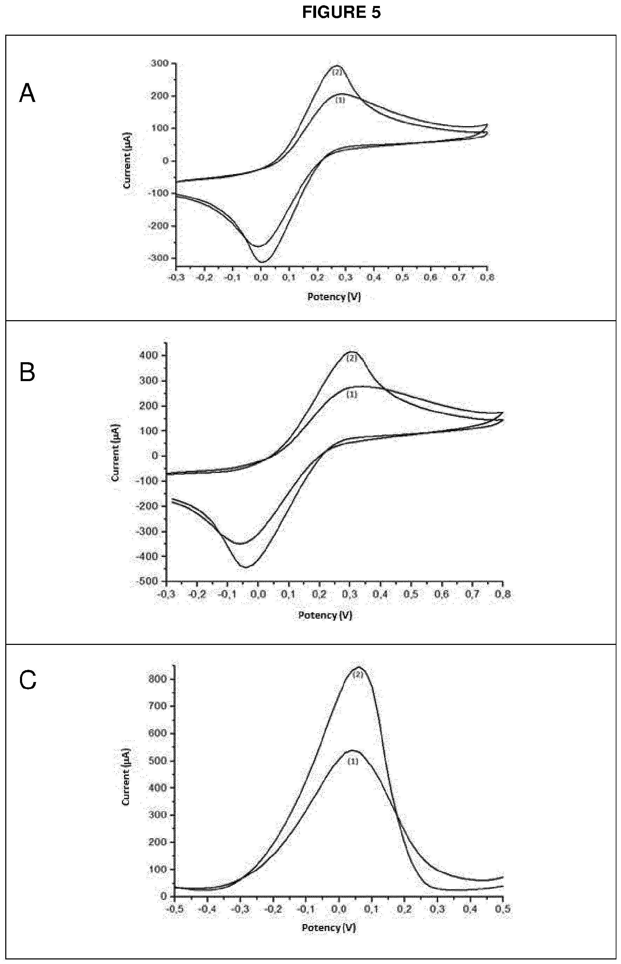

In FIGURE 5, clinical samples of dermal scrapings from patients and contacts suspected of leprosy were used. These were similarly immobilized on carbon electrodes modified with rhodamine 6G and anti-PGL-1 antibody. CURVES 1 refer to immobilization with positive dermal scraping and CURVES 2 with negative dermal scraping. It is observed that the curves of the positive samples have a lower oxidation and reduction current peak than the negative samples, since the antibody-antigen binding hinders the passage of electrons on the electrode surface, and when there is no specific binding, electron transfer is greater. In FIG. 5A, the test was performed in cyclic voltammetry with V= 200 mV.s-1 and showed differentiation between the current of the oxidation peaks of the positive and negative sample of 88µA. In FIG. 5B, however, the scan speed was increased to 500 mV.s-1 and the differentiation between samples also increased to 141µA at the oxidation peaks. In FIG 5C, the scanning curves were analyzed in differential pulse voltammetry and the difference between the oxidation currents of the positive and negative samples were even more significant (306µA).

### Study with other mycobacteria

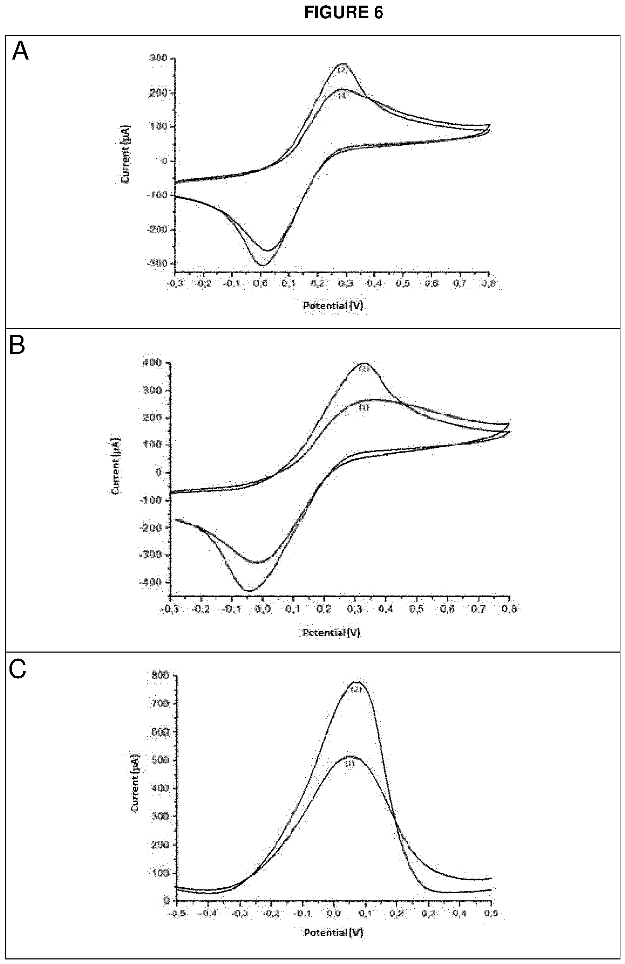

FIGURE 6 shows cyclic voltammetry (A and B) and differential pulse voltammetry (C) with the tuberculosis-specific antigen Anti-LAM immobilized on the modified electrode, as was done in the previous tests. In CURVES 1, the electrodes were incubated with TB culture (positive samples), whereas in CURVES 2 there was no target (negative control) being stored. There is a significant difference between the peaks of the oxidation currents of the positive and negative samples, 74µA in FIG. 6A, 139µA in FIG. 6B and 262µA in FIG. 6C. The results were similar to those of FIGURE 5, with clinical samples of dermal scraping due to the same form of recognition of the specific antibody, and the antigen with rhodamine 6G immobilized on bioelectrodes, thus confirming that this form of modification is useful in the diagnosis of other diseases and/or recognition of diverse biomolecules.

### Description of the invention

The electrode used was screen-printed graphite type, consisting of a working electrode (4 mm in diameter), a counter electrode and a reference electrode (Ref. DRP 110).

2µL to 4 µL of rhodamine 6G were applied on the working electrode surface, spread over the entire area (tested at different concentrations), and physical adsorption was done between 10 to 20 minutes at room temperature. Then, the electrode was connected to the receiver of the PalmSens 3 potentiostat (Compact Electrochemical Interfaces) and electrodeposition was performed in three consecutive measurements in cyclic voltammetry (V= 10 to 200 mV.s-1) using 50 to 120µL of potassium ferroferricyanide from 1 mM to 5mM / 0,1M Kcl as supporting electrolyte After this step, the electrode was washed with 100 µL de of distilled water and dried at room temperature.

Unmodified electrodes were also measured under the same conditions as above to determine the difference between them.

### Rhodamine 6G concentrations

To define the best rhodamine concentration to be used in the preparation of the electrode, variations from 100ug to of dye diluted in ultrapure water were tested. Cyclic voltammetry and differential pulse voltammetry with different scan speeds were performed to determine the active area of the electrode through the Randles-Sevcik equation. Biological antibody recognition tests were also performed at all concentrations.

### Detection of antigens on the surface of the modified electrode

For the formulation of the baseline, differential pulse voltammetry and cyclic voltammetry measurements at different scanning speeds on the modified and unmodified screen-printed electrode connected to a PalmSens 3 potentiostat (Compact Electrochemical Interfaces) were obtained, using 50 to 120µL of potassium ferroferricyanide from 1 mM to 5mM / 0.1M Kcl as supporting electrolyte.

In electrodes modified with rhodamine 6G, 2 to 10 µL of antibody specific for M. *leprae* (anti-PGL-1) or specific for M. *tuberculosis* (anti-LAM of TB) or other antibodies specific for mycobacteria and incubated at 25 to 37º for 5 to 50 minutes. Subsequently, they were incubated with 2 to 10 µL of antigen specific to native or synthetic antibodies for another 5 to 50 min from 25 to 37º and washed with distilled water. The readings were taken by differential pulse voltammetry and cyclic voltammetry in a portable potentiostat using potassium ferroferricyanide as supporting electrolyte.

### Cleaning and reusing electrodes

A technique for cleaning the used and modified electrodes with rhodamine was investigated, knowing that it is very soluble in organic solvents, ethyl alcohol was used, which can undo the bonds of such dye with the carbon on the surface of the electrodes. The electrodes used after adsorption of probes and targets were immersed in alcohol for 5 to 30 min and then washed with distilled water and dried at room temperature. Rhodamine 6G was again electrodeposited on said electrodes, preparing them for reuse.

### Detection in clinical samples

Dermal scrapes from patients and contacts suspected of leprosy were used. The slit skin technique assumes the collection of contaminated samples from the ear lobe, elbows, knees, and the active lesion if present, which are stored in phosphate buffer. The scrapes were previously quantified by real-time PCR and their concentrations were already known as well as samples classified as negative that were used as controls.

The *M. leprae* specific antibody (anti-PGL-1) was coupled to magnetic nanoparticles COFe₂O₄ with treatments for bioconjugation. After this process, the positive and negative scrape samples were incubated with the conjugated antibody for 30 min to 2 hours at 37 to 45ºC. The antigen-antibody conjugate was washed with PBS1X in a magnetic shelf and the part attached to the magnet was resuspended and applied 2 to 20 µL on the working area surface of the electrode modified with rhodamine 6G and incubated at a temperature between 25 to 37° for 5 to 50 minutes. The analyzes were performed by differential pulse voltammetry and cyclic voltammetry measurements at different scanning speeds on the electrode in a portable potentiostat and 50 to 120 µL of potassium ferroferricyanide from 1 mM to 5mM / 0.1 M Kcl was used as support electrolyte.

### Detection of other mycobacteria

A test was performed with electrodes modified with rhodamine 6G to detect another type of mycobacterium, *M. tuberculosis.* For this purpose, the specific antibody of this Anti-LAM tuberculosis pathogen was used, which was immobilized in the same way as in the previous tests, and for specific recognition, a tuberculosis culture sample was subsequently adsorbed in TLN medium (positive sample), and as a negative control only TLN medium without any type of pathogen was adsorbed and incubated at 25 to 37º for 5 to 50 minutes. The analyzes were performed by differential pulse voltammetry and cyclic voltammetry measurements at different scanning speeds on the electrode in a portable potentiostat and 50 to 120 µL of potassium ferroferricyanide from 1 mM to 5mM / 0.1 M Kcl were used as supporting electrolyte.

### Proposal for field use

Lyophilized kits containing anti-PGL1/anti-LAM or other antibodies specific for mycobacteria coupled or not to magnetic nanoparticles will be made available for subsequent agglutination, with various types of biological samples to be tested in the field that will be applied to electrodes already prepared with rhodamine 6G, which facilitates and speeds up the in-house diagnosis process.

### Conclusions

The present invention shows that rhodamine 6G worked as a versatile modifier of the transducer surface of commercial screen-printed electrodes. It was able to stabilize and functionalize the working area, as it easily adsorbs onto graphite carbon through the benzene ring and binds to proteins and other biological molecules through hydrogen bonds. Furthermore, its bond is easily undone when washed with ethyl alcohol, making the biosensor reusable The surface-modified immunosensor promoted the detection of antigens when specific antibodies were used as a target and differentiated dermal scraping samples from leprosy patients from contacts without the disease. It presented advantages inherent to the rapid diagnosis of these pathogens, applicability, specificity, sensitivity, stability, selectivity, and low cost. For example, this type of sensor can be used in the detection of biomolecules that have proteins or their biological or synthetic fragments, antigens, antibodies, peptides, DNA, RNA and aptamers, as analytes or as a biological recognition element.

In the process of modifying the surface of electrodes for the construction of biosensors, graphite working electrode (screen-printed DRP 110) or other working electrodes, preferably made of conductive material, presenting electrochemical inertia in the range, can be used of -0,4V and +1,4V (versus Ag/AgCl or Ag), such as glassy carbon, carbon paste, diamond, gold, platinum, and may be a combination of nanotechnological materials such as polymeric films, graphene, carbon nanotubes and nanoparticles on the surface of electrodes as well as probes used for recognition.

Rhodamine 6G was used in concentrations ranging from 100µg to 100mg, diluted in organic solvent, and surfactants, chemical compounds or nanotechnological materials can be added to improve the bond with the surface of the electrodes and with the biomolecules.

Specific antibodies and their respective native antigens, mimetics, bacterial cultures, and samples of patient swabs can be used, not restricted to use only for mycobacteria, but for immunosensors in general using biomolecules that have proteins or their biological fragments or synthetics, antigens, antibodies, peptides, DNA, RNA, and aptamers, as analytes or as a biological recognition element.

The great advantage of the present invention is to make the biosensor reusable by washing the modified electrode with organic solvent at different concentrations.

The modification of electrodes and detection of biological materials occurs through electrochemical analysis, following the variation of signals by differential pulse voltammetry, cyclic voltammetry, square wave voltammetry or other appropriate electrochemical technique, of the oxidation peak or reduction of the specific recognition binding between probe and targets.

The modification of the transducer surface of electrodes with rhodamine 6g may be responsible for obtaining an electrochemical sensor that will be used in the detection of biomolecules that have proteins or their biological or synthetic fragments, antigens, antibodies, peptides, DNA, RNA enzymes and aptamers, as analytes or as a biological recognition element.

## Claims

1. An electrode surface modification process for construction of electrochemical biosensors **characterized in that** it comprises the following steps:
a) 2µL to 4 µL of rhodamine 6G were applied to the working electrode surface;
b) physical adsorption was carried out between 10 to 20 minutes at room temperature;
c) then, the electrode was connected to the receiver of the PalmSens 3 potentiostat (Compact Electrochemical Interfaces) and electrodeposition was performed in three consecutive measurements in cyclic voltammetry (V= 10 to 200 mV.s-1) using 50 to 120 µL of potassium ferroferricyanide from 1 mM to 5mM / 0.1M Kcl as supporting electrolyte;
d) after this step, the electrode was washed with 100 µL of distilled water and then dried at room temperature.

2. The modification process as in claim 1, **wherein** it comprises a graphite electrode (screen-printed DRP 110) or other working electrodes preferably of conductive material, presenting electrochemical inertia in the range of -0.4V and +1.4V (versus Ag/AgCl or Ag), such as vitreous carbon, carbon paste, diamond, gold, platinum, and may be a combination of nanotechnological materials such as polymeric films, graphene, carbon nanotubes and nanoparticles on the surface of the electrodes, as well as in the probes used for recognition.

3. The modification process as in claim 1, **wherein it** encompasses the use of rhodamine 6G in concentrations ranging from 100µg to 100mg diluted in an organic solvent, and surfactants, chemical compounds or nanotechnological materials can be added to improve the bond with the surface of the electrodes and with the biomolecules.

4. The modification process as in claim 1, **wherein it** encompasses the use of specific antibodies and their respective native antigens, mimetics, bacterial cultures and samples of patient scrapes, not being restricted to use only in mycobacteria, but for immunosensors in general, using biomolecules that have proteins or their biological or synthetic fragments, antigens, antibodies, peptides, DNA enzymes, RNA and aptamers, as analytes or as a biological recognition element.

5. The modification process as in claim 1, **wherein it** makes the biosensor reusable by washing the modified electrode with organic solvent at different concentrations.

6. The modification process as in claim 1, **characterized by** being the modification of electrodes and the detection of biological materials through electrochemical analysis, following the variation of signals by differential pulse voltammetry, cyclic voltammetry, square wave voltammetry or other appropriate electrochemical technique, of the oxidation peak or reduction of the binding of specific recognition between probe and targets.

7. The modification process as in claim 1, **characterized in that** the modification of the transducer surface of electrodes with rhodamine 6g is responsible for obtaining an electrochemical sensor that can be used in the detection of biomolecules that have proteins or their biological or synthetic fragments, antigens, antibodies, peptides, DNA enzymes, RNA, and aptamers, as analytes or as a biological recognition element.
